(19) Europäisches Patentamt / European Patent Office / Office européen des brevets

(11) **EP 3 557 528 B1**

(12) **FASCICULE DE BREVET EUROPEEN**

(45) Date de publication et mention
de la délivrance du brevet:
**13.07.2022 Bulletin 2022/28**

(21) Numéro de dépôt: **19169463.7**

(22) Date de dépôt: **16.04.2019**

(51) Classification Internationale des Brevets (IPC):
**G06T 7/33** (2017.01)    **G06T 5/00** (2006.01)
**C12M 1/34** (2006.01)    **G01N 21/78** (2006.01)

(52) Classification Coopérative des Brevets (CPC):
**G06T 7/33; C12M 41/36; G06T 5/006;** G01N 21/78;
G06T 2201/0051; G06T 2207/10008

(54) **SYSTEME ET PROCEDE D'ANALYSE DE DISPOSITIFS DE TEST**

SYSTEM UND VERFAHREN ZUR ANALYSE VON TESTVORRICHTUNGEN

SYSTEM AND METHOD FOR ANALYSING TEST DEVICES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.04.2018 FR 1853321**

(43) Date de publication de la demande:
**23.10.2019 Bulletin 2019/43**

(73) Titulaire: **Pinqkerton**
**67300 Schiltigheim (FR)**

(72) Inventeur: **PEASE, Christopher**
**67300 SCHILTIGHEIM (FR)**

(74) Mandataire: **Santarelli**
**49, avenue des Champs-Elysées**
**75008 Paris (FR)**

(56) Documents cités:
**EP-A1- 2 453 242        WO-A1-98/14777
WO-A1-2013/116831    WO-A1-2014/099643
WO-A2-2014/025415    WO-A2-2014/080212
WO-A2-2016/172527    US-A1- 2015 241 358
US-A1- 2016 048 739    US-A1- 2017 067 832**

**Description**

La présente invention concerne un système et un procédé d'analyse de dispositifs de test, notamment de tests biologiques ou chimiques.

En particulier, la présente invention peut s'appliquer à des dispositifs de test comprenant des milieux de croissance biologique recevant par exemple des échantillons d'aliments, des échantillons de ressources d'eau, d'eaux traitées ou de rejets, de carburants ou encore des échantillons environnementaux, de laboratoire ou d'hôpitaux. Après incubation, des colonies de micro-organismes se développent sur le milieu de croissance. Ces colonies sont comptées et classifiées. Une fois le nombre et les types de colonies déterminés, il est possible d'établir un diagnostic, ce dernier pouvant alors être utilisé pour, par exemple, appliquer des mesures correctives visant à améliorer la sécurité biologique, des rendements de production ou la qualité de produits. Il est noté ici que les tests de contamination biologique sont devenus très importants et obligatoires dans de nombreux domaines.

Diverses techniques ou appareils peuvent être utilisés pour analyser des résultats des tests afin d'améliorer et augmenter la répétabilité, la fiabilité et la traçabilité de l'analyse de ces tests.

Ainsi, à titre d'illustration, un échantillon, par exemple liquide, peut être placé sur un milieu de croissance biologique d'un dispositif de test, le dispositif de test étant ensuite inséré dans une chambre d'incubation puis, après incubation, introduit dans un scanner pour analyser la croissance de colonies et les dénombrer automatiquement, réduisant les erreurs d'interprétation.

La demande de brevet WO 2014/080212 décrit un exemple de bandelettes réactives ainsi qu'un procédé et un dispositif de lecture permettant d'effectuer des analyses à partir d'images des bandelettes. La demande de brevet WO 2016/172527 concerne un appareil pour évaluer l'évolution de colonies par analyse d'images, en fonction du contraste, de la couleur et du temps.

La **figure** 1 représente un système 10 de test et d'analyse selon l'enseignement du brevet US 7,298,885. Le système 10 comprend un scanner 12, un écran 14 et un support 16 pour recevoir un dispositif de test 20 et l'introduire dans le corps du scanner 12. Le dispositif 20 a une zone de test 22 et un identifiant 24 qui permet d'identifier le dispositif 20 (en particulier déterminer l'analyse à effectuer). Le scanner 12 peut alors identifier le type de dispositif 20 grâce à l'identifiant 24 et appliquer automatiquement une analyse appropriée. L'écran 14 permet, par exemple, d'afficher la zone de test 22, la progression de l'analyse (le temps restant), des options sélectionnables par un utilisateur (par exemple des paramètres d'analyse) et/ou les résultats de l'analyse du dispositif 20.

Cependant, bien que répondant à des problématiques, le système 10 présente des inconvénients, notamment en termes de coûts, de versatilité (ce système n'est adapté qu'à un type de dispositif spécifique) et de mise en œuvre (ce système n'est pas adapté à une utilisation sur le terrain).

Il existe donc un besoin pour un système et un procédé d'analyse de dispositifs de test, biologiques ou chimiques, capable d'être mis en œuvre facilement et à moindre coût, même dans un environnement à faibles ressources, c'est à dire sans environnement contrôlé ni personnel formé aux techniques aseptiques.

Des modes de réalisation de l'invention concernent un procédé d'analyse d'un dispositif de test comme visé dans les revendications 1 à 5.

Le procédé selon l'invention permet ainsi d'analyser facilement et à moindre coût des dispositifs de test, par exemple des dispositifs de test biologiques ou chimiques, en particulier dans des environnements non contrôlés et/ou en l'absence de personnel qualifié.

L'invention a également pour objet un programme d'ordinateur comprenant des instructions adaptées à la mise en œuvre de chacune des étapes du procédé décrit précédemment, lorsque ledit programme est exécuté sur un ordinateur. Les avantages procurés par ce programme d'ordinateur sont similaires à ceux évoqués précédemment.

L'invention a également pour objet un système d'analyse d'un dispositif de test comprenant des moyens adaptés à la mise en œuvre de chacune des étapes du procédé décrit précédemment. Les avantages procurés par ce système sont similaires à ceux évoqués au regard du procédé.

Selon des modes de réalisation, le système comprend un dispositif de capture d'image, de correction d'image et d'analyse d'images de type smartphone.

Selon des modes de réalisation, le système comprend un premier dispositif de capture d'image et un second dispositif d'analyse d'images, distinct du premier dispositif.

D'autres avantages, buts et caractéristiques de la présente invention ressortent de la description détaillée qui suit, faite à titre d'exemple non limitatif, au regard des dessins annexés dans lesquels :

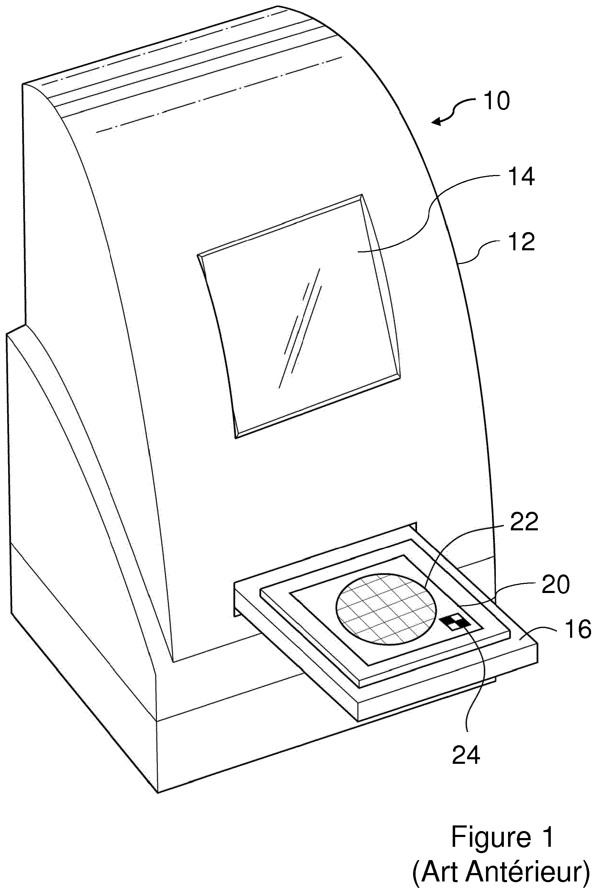

- la figure 1 représente un système connu d'analyse de dispositifs de test ;
- les figures 2A et 2B représentent des vues de face d'un dispositif de test pouvant être utilisé par un système d'analyse, selon des modes de réalisation, dans deux configurations différentes ;
- la figure 3 représente une vue en coupe, de profil, du dispositif illustré sur les figures 2A et 2B, dans la configuration de la figure 2B ;

- les figures 4A, 4B, 4C et 4D illustrent des observations successives d'un même dispositif de test au cours de tests ;
- les figures 5A, 5B et 5C illustrent un premier exemple de normalisation basée sur un format prédéterminé d'une référence visuelle afin de permettre la comparaison d'images d'un dispositif de test obtenues de plusieurs points de vue différents ;
- les figures 6A, 6B et 6C illustrent un second exemple de normalisation basée sur un format prédéterminé d'une référence visuelle afin de permettre la comparaison d'images d'un dispositif de test obtenues de plusieurs points de vue différents ;
- les figures 7A et 7B illustrent un exemple de correction d'images selon un mode de réalisation particulier ;
- la figure 8 représente un exemple d'une unité d'analyse pouvant être utilisée par un système d'analyse selon un mode de réalisation particulier ;
- la figure 9 illustre des étapes d'un exemple de procédé d'analyse selon un mode de réalisation ;
- la figure 10 représente un exemple de système d'analyse selon un mode de réalisation particulier ; et
- la figure 11 représente une vue de face d'un dispositif de test pouvant être utilisé par un système selon des modes de réalisation.

D'une manière générale, l'invention concerne un système et un procédé d'analyse de dispositifs de tests, notamment de tests biologiques ou chimiques, permettant par exemple la détection, le dénombrement et/ou la classification de colonies de micro-organismes, utilisant une même référence visuelle pour identifier le dispositif (permettant, de préférence, de sélectionner un type d'analyse à effectuer) et pour analyser le dispositif par analyse d'images, sans nécessiter un point de vue particulier lors de la prise d'images du dispositif.

Des modes de réalisation de l'invention sont adaptés à l'observation de la croissance de colonies de micro-organismes afin d'en analyser l'évolution au fil du temps.

Dans des modes de réalisation particuliers, une unité d'analyse, telle qu'un téléphone intelligent (smartphone) ou une tablette équipée d'un capteur d'image, comprenant une application logicielle adaptée, peut être utilisée pour capturer une image du dispositif, les images capturées pouvant être traitées et analysées soit par l'unité d'analyse elle-même (si elle a les ressources nécessaires) soit, par exemple, par un serveur distant auquel sont transférées les images ou des parties d'images. Lorsque le traitement et l'analyse sont effectués dans un dispositif distant, l'unité d'analyse peut consister en un simple dispositif de prise de vues numériques.

Toujours dans des modes de réalisation particuliers, le procédé d'analyse est mis en oeuvre dans l'unité d'analyse, à l'aide d'une application adaptée, qui est utilisée pour la capture d'images, la normalisation des images acquises et leur analyse. L'utilisateur peut en outre être guidé dans l'utilisation de l'application, par exemple pour indiquer les instants auxquels des images doivent être prises.

Le dispositif de test est maintenant décrit plus en détail.

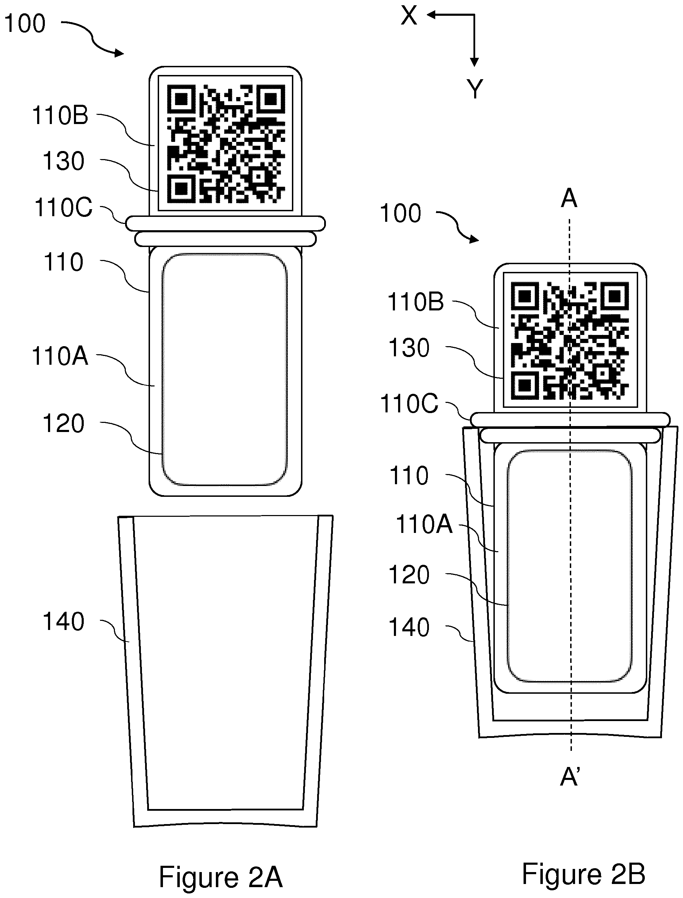

Les **figures 2A** et **2B** représentent des vues de face d'un même dispositif de test 100 pouvant être utilisé par un système d'analyse, selon des modes de réalisation, dans deux configurations différentes.

A titre d'illustration, les modes de réalisation de l'invention décrits ci-dessous visent un dispositif de croissance biologique de colonies de micro-organismes. Cependant, l'invention n'est pas limitée à ce domaine.

Le dispositif de test 100 peut prendre une configuration ouverte, comme illustré sur la figure 2A ou une configuration fermée comme illustré sur la figure 2B.

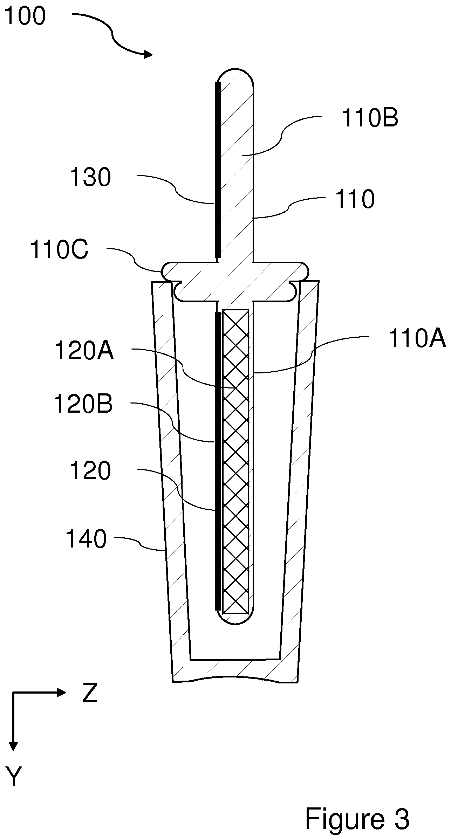

La **figure 3** représente une vue en coupe, de profil, du dispositif de test 100 illustré sur les figures 2A et 2B, dans la configuration de la figure 2B.

Comme illustré, le dispositif de test 100 comprend un support 110, une zone de test 120, une référence visuelle 130 et une enveloppe de protection 140. La référence visuelle 130 permet une identification du dispositif de test 100 et une normalisation d'une image représentant ce dispositif, typiquement par la détermination d'une déformation géométrique d'une représentation de la zone de test en fonction d'un format prédéterminé de la référence visuelle.

Le support 110 comprend une première partie 110A, appelée partie inférieure, et une seconde partie 110B, appelée partie supérieure, séparées par une butée de fermeture 110C. La partie inférieure 110A comprend la zone de test 120. Elle est configurée pour pouvoir être insérée dans l'enveloppe de protection 140, comme montré sur les figures 2B et 3. Il est observé ici que la butée de fermeture 110C peut être une pièce rapportée.

L'enveloppe de protection 140 forme, avec le support 110, un système clos enveloppant et isolant la zone de test 120 de l'environnement extérieur. L'enveloppe de protection 140 est idéalement transparente afin de permettre des captures d'image régulières au cours de l'essai, sans exposer la zone de test 120 à l'air ambiant et, ainsi, à une possible contamination extérieure.

La partie supérieure 110B peut être utilisée pour la manipulation du dispositif de test 100 sans qu'un utilisateur ne touche la zone de test 120 et risque de la contaminer. La référence visuelle 130 est ici agencée sur la partie supérieure 110B, idéalement au-dessus de la butée 140 et en dehors de l'enveloppe de protection 140 afin que sa lecture ne soit pas perturbée.

Comme illustré sur la figure 3 représentant une vue en coupe selon l'axe A-A' de la figure 2B (i.e. selon un plan YZ où l'axe Y est orienté selon la longueur du dispositif 100 et l'axe Z est orienté selon sa profondeur), la zone de test 120 comprend ici un milieu de développement 120A, par exemple un milieu nutritif, et une surface d'observation 120B. Le milieu de développement 120A permet par exemple le développement de micro-organismes et/ou de marqueurs chimiques et/ou biologiques permettant de révéler la présence d'activités biologiques des micro-organismes et/ou permettant de dénombrer et/ou classer ces micro-organismes.

Selon des modes de réalisation, la zone de test 120 et la référence visuelle 130 sont agencées sur la même face du support 110, de préférence dans le même plan XY (l'axe X étant orienté selon la largeur du dispositif 100 et l'axe Y étant orienté selon la longueur du dispositif 100) ou dans des plans parallèles.

La surface d'observation 120B recouvre, de préférence, le milieu de développement 120A qui est, par exemple, plan et de forme carrée ou circulaire. Toujours à titre d'illustration, le milieu de développement 120A peut comprendre un support tel qu'un tampon pouvant être constitué de dérivés cellulosiques pour retenir un liquide ou un gel permettant le développement de micro-organismes.

L'enveloppe de protection 140 peut servir de contenant pour recevoir (de préférence temporairement) un échantillon à analyser dans lequel la zone de test 120 peut être plongée.

Après que la zone de test 120 ait été mise en contact avec un échantillon à tester et, le cas échéant, que l'excédent d'échantillon ait été retiré de la zone de test 120, le dispositif de test 100 est laissé incuber pendant un certain temps, la zone de test 120 étant protégée dans l'enveloppe de protection 140. Comme décrit ci-après, des photographies du dispositif peuvent être prises et analysées selon la nature des tests à effectuer.

La référence visuelle 130 est, par exemple, un identifiant visuel du type QR code (sigle de *Quick Response code* en terminologie anglo-saxonne), un code-barres ou un autre identifiant ayant un format prédéterminé. Ce format peut être défini, par exemple, par le fabricant du dispositif, le développeur d'un logiciel de traitement d'images selon des modes de réalisation de l'invention ou par un laboratoire d'analyse qui reçoit des images du dispositif de test.

La référence visuelle 130 permet typiquement de déterminer une analyse à effectuer. Cette référence peut être commune à plusieurs dispositifs de test ou propre à chaque dispositif de test.

L'identifiant fourni par la référence visuelle 130, qu'il soit unique ou commun à plusieurs dispositifs de test, peut être utilisé comme une entrée de base de données afin d'obtenir et/ou renseigner des informations telles qu'un type de micro-organisme, un fabricant, un protocole de test, une date de validité, le nom d'un utilisateur, une date d'utilisation, etc. Cette base de données peut être initialement complétée par le fabricant du dispositif de test et enrichie ensuite, lorsque le dispositif de test est utilisé. Plusieurs bases de données, correspondant à plusieurs dispositifs de test et/ou à plusieurs types d'informations, peuvent être utilisées.

La référence visuelle 130 permet en outre de normaliser, si nécessaire, une image représentant le dispositif de test en adaptant, par exemple, le facteur d'échelle et/ou la perspective. A ces fins, la référence visuelle 130 est utilisée comme référence pour déterminer une déformation ou pour définir une telle référence.

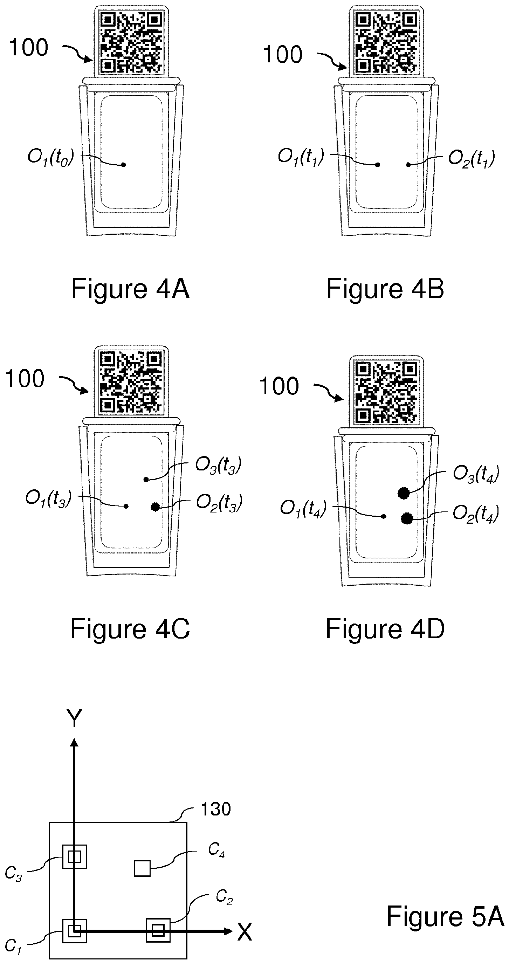

Les **figures 4A, 4B, 4C et 4D** illustrent des observations successives d'un même dispositif de test au cours de tests.

La figure 4A illustre le dispositif de test 100 à un temps $t_0$, par exemple juste après que la zone de test 120 du dispositif 100 ait été mise en contact avec l'échantillon à analyser. Comme illustré sur la figure 4A, une tâche notée $O_1(t_0)$ est observée sur la surface d'observation 120B à l'instant $t_0$. Les caractéristiques de cette tâche, par exemple sa position, sa taille, sa couleur et sa forme, sont, de préférence, enregistrées pour permettre d'analyser son évolution.

La figure 4B illustre le même dispositif de test 100 à un temps $t_1$, par exemple 24h après le temps $t_0$. A nouveau, les caractéristiques des tâches observées, par exemple sa position, sa taille, sa couleur et sa forme, peuvent être enregistrées. Selon l'exemple illustré sur la figure 4B, la tâche $O_1$ n'a pas évolué entre les instants $t_0$ et $t_1$ (i.e. les tâches $O_1(t_0)$ et $O_1(t_1)$ sont équivalentes) et la tâche $O_2$ est apparue sur la surface d'observation 120B.

Comme illustré sur la figure 4C, représentant le même dispositif de test 100, la surface d'observation 120B montre, à un instant $t_2$, par exemple 48h après l'instant $t_0$, les tâches $O_1$ et $O_2$ déjà observées précédemment et une nouvelle tâche $O_3$. La taille de la tâche $O_2$ a ici augmenté par rapport à l'observation effectuée à l'instant $t_1$. A nouveau encore, les caractéristiques des tâches observées, par exemple sa position, sa taille, sa couleur et sa forme, sont ici enregistrées.

Toujours à titre d'illustration, la figure 4D illustre le même dispositif 100 à un instant $t_3$, par exemple 72h après l'instant $t_0$. Selon cet exemple, aucune nouvelle tâche n'est apparue et la taille des tâches $O_2$ et $O_3$ a augmenté par rapport à l'observation effectuée à l'instant $t_2$, la taille de la tâche $O_3$ ayant augmenté plus rapidement que celle de la tâche $O_2$. A nouveau encore, les caractéristiques des tâches observées, par exemple sa position, sa taille, sa couleur et sa forme, sont, de préférence, enregistrées.

Il est noté ici que les caractéristiques de la tâche $O_1$ n'ont pas évoluées entre les instants $t_0$ à $t_3$. Il peut donc en être conclu que la tâche $O_1$ n'est pas représentative d'une colonie de micro-organisme. Il peut s'agir, par exemple, d'une poussière. Par ailleurs, il peut être considéré que la tâche $O_2$ représente une colonie de micro-organismes à croissance lente (dans les conditions de test) et que la tâche $O_3$ représente une colonie de micro-organismes à croissance rapide (toujours dans les conditions de test).

De façon générale, les colonies de micro-organismes apparaissent dans des « vagues » de temps s'il y a des sous-populations dans l'échantillon analysé. Si les captures d'images sont assez fréquentes, la détection de telles vagues peut être une indication du nombre de sous-populations, étant observé qu'il faut environ un million de micro-organismes pour qu'une colonie soit observable à l'œil nu mais que seulement quelques milliers de micro-organismes peuvent généralement être détectés par un grossissement x10 (souvent proposé par les capteurs d'images d'un smartphone ou d'une tablette).

La vitesse à laquelle la taille d'une colonie se développe est une indication du temps de doublement (en phase exponentielle) constituant une indication de la souche spécifique de la colonie, étant observé qu'entre l'instant initial et la phase exponentielle, les bactéries connaissent généralement une phase dite de latence durant laquelle elles s'adaptent à leur nouvel environnement et ne se multiplient pas ou peu.

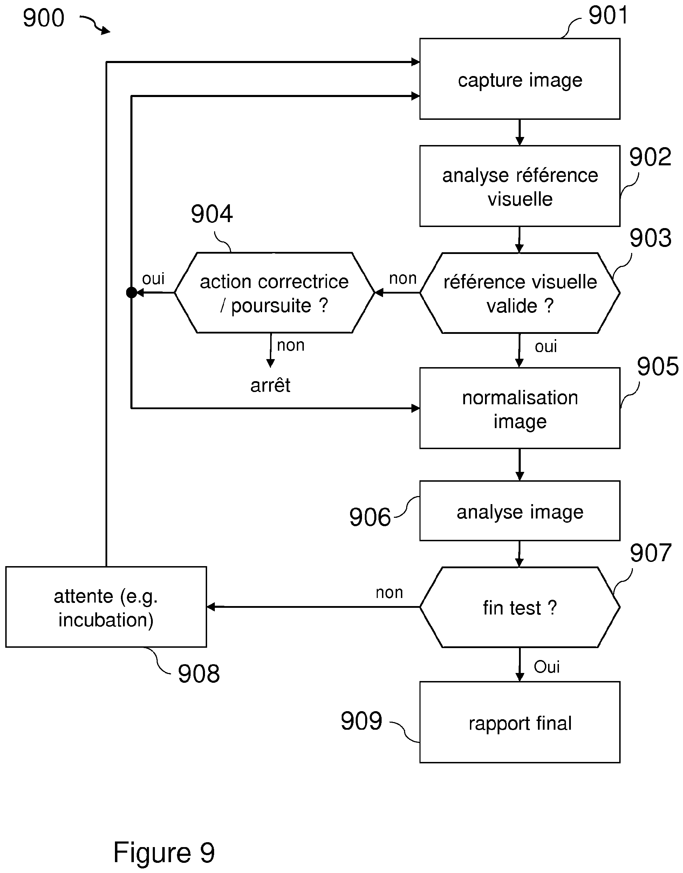

Les colonies sont généralement rondes mais peuvent être tronquées, se chevaucher, ou avoir des formes différentes (irrégulières, filamenteuses, rhizoïdes, etc.) selon le type de colonie. En outre, la couleur d'une colonie est généralement blanche mais peut être transparente, de couleur coquille d'œuf, ou autre. En outre, les milieux nutritifs peuvent générer des couleurs de colonie distinctes ou des halos autour des colonies. Ces aspects sont bien connus dans l'art. L'analyse d'une image peut comprendre alors l'évaluation de la forme, de la couleur, de la bordure, etc. D'autres techniques optiques permettent d'observer le relief et la texture des colonies, par exemple par analyse de la déformation d'un motif connu projeté sur et réfléchi par la zone de test 120. Par un simple système de miroir, il est possible de saisir le motif réfléchit et la référence visuelle 130 sur une même image, puis d'effectuer une analyse (comme décrit par exemple en référence aux étapes 902, 905 et 906 de la figure 9). Les informations de relief et de textures sont complémentaires des informations sur la taille, vitesse de croissance, couleur, transparence et similaires et augmentent la capacité du système à classifier correctement les colonies.

Selon des modes de réalisations, l'utilisateur est sollicité pour capturer les images qui sont ensuite analysées. Il peut ainsi être alerté au début du test du nombre d'images à prendre et des instants auxquels les images doivent être prises. Il peut en outre être averti chaque fois qu'une image doit être prise.

Comme décrit précédemment, la référence visuelle 130 permet, suite à la capture d'une image

- d'identifier un dispositif de test 100 et, ainsi, obtenir des caractéristiques sur ce dispositif de test et/ou sur des tests en cours, effectués ou à effectuer ;
- d'identifier une partie d'image contenant au moins une portion d'une surface d'observation 120B à analyser ;
- d'établir un lien entre plusieurs images acquises pour étudier une évolution d'une surface d'observation ; et/ou
- d'établir une correspondance entre des points d'une surface d'observation de plusieurs images, sans nécessiter l'utilisation d'un point de vue prédéterminé.

A ces fins, la référence visuelle 130 permet notamment l'exécution d'une étape de normalisation afin que des images prises de différents points de vue puissent être comparées. Il est ainsi possible de normaliser les captures d'images par la connaissance du format de la référence visuelle 130.

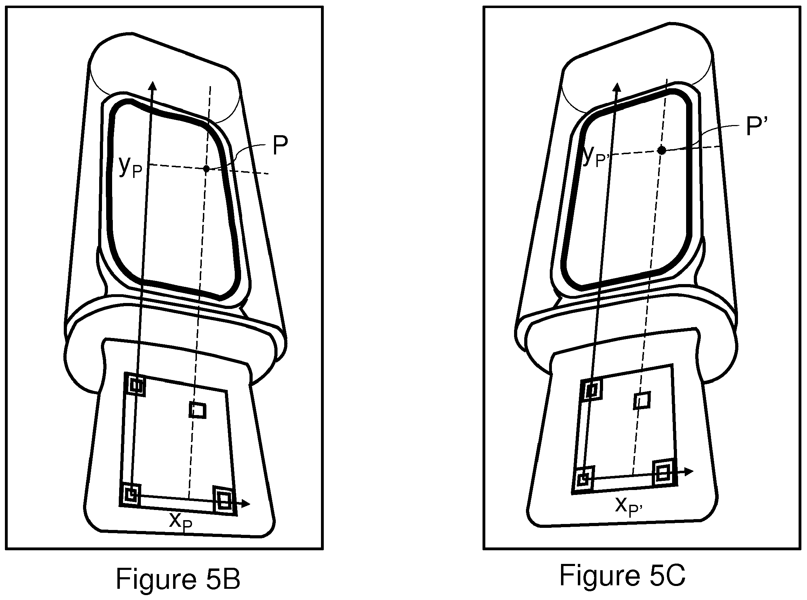

Les **figures 5A, 5B et 5C** illustrent un premier exemple de normalisation basée sur un format prédéterminé d'une référence visuelle afin de permettre la comparaison d'images d'un dispositif de test obtenues de plusieurs points de vue différents.

Selon ce mode de réalisation, il est admis que la référence visuelle et la surface d'observation du dispositif de test sont disposées dans un même plan.

La figure 5A illustre un exemple de référence visuelle utilisée comme repère géométrique. La référence visuelle 130 utilisée est ici un QR code ayant une taille prédéterminée. Le QR code comprend trois carrés distincts $C_1$, $C_2$ et $C_3$, appelés carrés de position, disposés dans les coins inférieur gauche, inférieur droit et supérieur gauche, respectivement, comme illustré. Le QR code comprend en outre un quatrième carré $C_4$, appelé carré d'alignement (le QR code peut comprendre plusieurs carrés d'alignement selon la version du code QR mise en oeuvre). Le ou les carrés d'alignement permettent de corriger une image du code par rapport à la taille, l'orientation et l'angle de prise d'image.

Le centre des carrés de position $C_1$, $C_2$ et $C_3$ est utilisé pour former un repère géométrique dans lequel peuvent être exprimées les coordonnées des points de la référence visuelle ainsi que les coordonnées des points de la surface d'observation du dispositif de test (la référence visuelle et la surface d'observation étant ici placées dans un même plan). Comme illustré sur la figure 5A, le centre du carré de position $C_1$ forme l'origine du repère, le centre des carrés de position $C_1$ et $C_2$ forme l'axe des abscisses ainsi que le vecteur unité selon cet axe et le centre des carrés de position $C_1$ et $C_3$ forme l'axe des ordonnées ainsi que le vecteur unité selon cet axe.

Les figures 5B et 5C illustrent deux images représentant un dispositif de test 100 observé depuis deux points de vue distincts.

Comme illustré, l'identification de la référence visuelle 130 et des carrés de position $C_1$, $C_2$ et $C_3$, selon des techniques d'analyse d'image standard utilisées, par exemple, pour identifier des QR codes, permet de définir un repère géométrique lié à la référence visuelle 130, comme décrit en référence à la figure 5A. Les coordonnées de points de la surface d'observation du dispositif de test analysé, issus d'images obtenues selon des points d'observation différents, par exemple les point *P* et *P'* sur les figures 5B et 5C, dans ce repère « normalisé », peuvent être comparées les unes aux autres pour étudier, par exemple, le développement de colonies de micro-organismes.

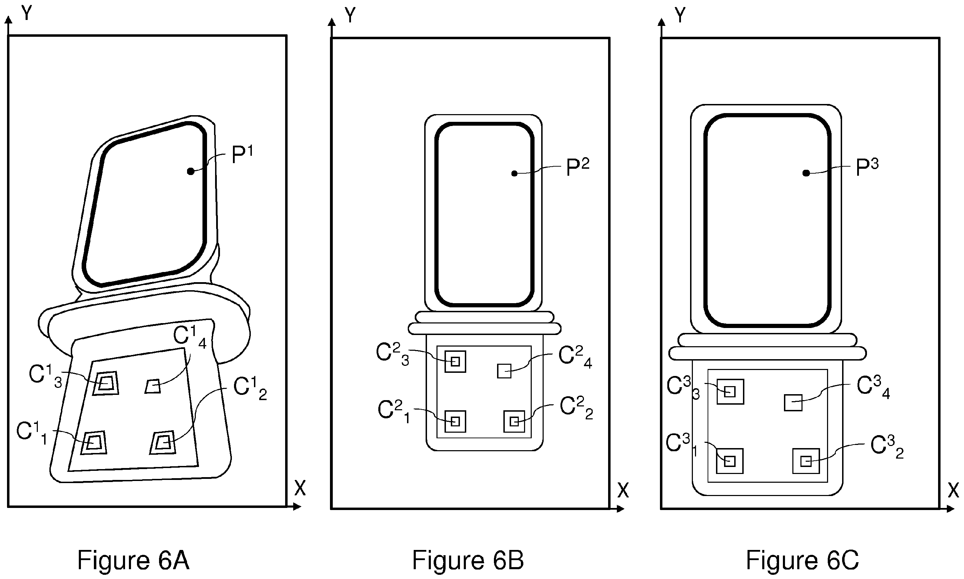

Les **figures 6A, 6B et 6C** illustrent un second exemple de normalisation basée sur un format prédéterminé d'une référence visuelle afin de permettre la comparaison d'images d'un dispositif de test obtenues de plusieurs points de vue différents.

La référence visuelle utilisée ici est similaire à celle illustrée sur la figure 5A.

Les figures 6A, 6B et 6C montrent trois images représentant un même dispositif de test observé selon trois points de vue différents à trois instants différents.

Il est ici considéré un repère orthonormé lié aux images analysées. A titre d'illustration, il est considéré ici que l'origine du repère est le coin inférieur gauche des images et que l'unité est le pixel.

A nouveau, des techniques d'analyse d'image standard mises en oeuvre, par exemple, pour identifier des QR codes, peuvent être utilisées pour identifier les carrés de position et d'alignement $C_1$, $C_2$, $C_3$ et $C_4$ et déterminer les coordonnées de leur centre, noté $(x_1, y_1)$, $(x_2, y_2)$, $(x_3, y_3)$ et $(x_4, y_4)$, respectivement, dans un repère lié à l'image dans laquelle sont identifiés ces carrés.

En déterminant la relation entre chaque paire de points formée par ces quatre points dans deux images différentes, il est possible d'en déduire la projection d'une image vers l'autre et, par conséquent, d'analyser l'évolution de tâches identifiées dans une surface d'observation.

La figure 6A représente une première image 600 d'un dispositif de test obtenue selon un premier point de vue, dans laquelle il est possible d'identifier les carrés de position et d'alignement $C^1_1$, $C^1_2$, $C^1_3$ et $C^1_4$ et déterminer les coordonnées de leur centre, noté $(x^1_1, y^1_1)$, $(x^1_2, y^1_2)$, $(x^1_3, y^1_3)$ et $(x^1_4, y^1_4)$, respectivement, dans un repère lié à l'image dans laquelle sont identifiés ces carrés. L'image 600 a par exemple été obtenue à un instant $t_1$.

De même, la figure 6B représente une seconde image 605 du dispositif de test obtenue selon un deuxième point de vue, dans laquelle il est possible d'identifier les carrés de position et d'alignement $C^2_1$, $C^2_2$, $C^2_3$ et $C^2_4$ et déterminer les coordonnées de leur centre, noté $(x^2_1, y^2_1)$, $(x^2_2, y^2_2)$, $(x^2_3, y^2_3)$ et $(x^2_4, y^2_4)$, respectivement, dans un repère lié à l'image dans laquelle sont identifiés ces carrés. L'image 605 a par exemple été obtenue à un instant $t_2$.

De même encore, la figure 6C représente une troisième image 610 du dispositif de test obtenue selon un troisième point de vue, dans laquelle il est possible d'identifier les carrés de position et d'alignement $C^3_1$, $C^3_2$, $C^3_3$ et $C^3_4$ et déterminer les coordonnées de leur centre. L'image 610 a par exemple été obtenue à un instant $t_3$.

A partir des coordonnées $(x^1_1, y^1_1)$, $(x^1_2, y^1_2)$, $(x^1_3, y^1_3)$ et $(x^1_4, y^1_4)$ et des coordonnées $(x^2_1, y^2_1)$, $(x^2_2, y^2_2)$, $(x^2_3, y^2_3)$ et $(x^2_4, y^2_4)$, il est possible de déterminer la projection de n'importe quel point de l'image 600 dans l'image 605 et inversement.

Ainsi, par exemple, comme décrit dans l'article intitulé « Perspective Correction Methods for Camera-Based Document Analysis », L. Jagannathan and C. V. Jawahar, Proceedings of First International Workshop on Camera Based Document Analysis and Récognition, August 2005, Seoul, Korea pp 148-154, les coordonnées $(x^2_p, y^2_p)$ du point $P^2$ de l'image 605 peuvent s'exprimer à partir des coordonnées $(x^1_p, y^1_p)$ du point $P^1$ de l'image 600 et d'une homographie H caractérisée par la matrice homogène 3x3 suivante

$$\begin{bmatrix} h_{11} & h_{12} & h_{13} \\ h_{21} & h_{22} & h_{23} \\ h_{31} & h_{32} & h_{33} \end{bmatrix}$$

où le coefficient h33 peut être fixé à la valeur 1,

$$x^2_p = \frac{h_{11}x^1_p + h_{12}y^1_p + h_{13}}{h_{31}x^1_p + h_{32}y^1_p + 1}$$

$$y^2_p = \frac{h_{21}x^1_p + h_{22}y^1_p + h_{23}}{h_{31}x^1_p + h_{32}y^1_p + 1}$$

les coefficients de l'homographie pouvant par exemple être calculés à partir des points $C^1_1$, $C^1_2$, $C^1_3$ et $C^1_4$ et $C^2_1$, $C^2_2$, $C^2_3$ et $C^2_4$, par la résolution du système d'équation suivant,

$$\begin{bmatrix} x_1^1 & y_1^1 & 1 & 0 & 0 & 0 & -x_1^1 x_1^2 & -y_1^1 x_1^2 \\ x_1^1 & y_1^1 & 1 & 0 & 0 & 0 & -x_2^1 x_2^2 & -y_2^1 x_2^2 \\ x_1^1 & y_1^1 & 1 & 0 & 0 & 0 & -x_3^1 x_3^2 & -y_3^1 x_3^2 \\ x_1^1 & y_1^1 & 1 & 0 & 0 & 0 & -x_4^1 x_4^2 & -y_4^1 x_4^2 \\ 0 & 0 & 0 & x_1^1 & y_1^1 & 1 & -x_1^1 y_1^2 & -y_1^1 y_1^2 \\ 0 & 0 & 0 & x_1^1 & y_1^1 & 1 & -x_2^1 y_2^2 & -y_2^1 y_2^2 \\ 0 & 0 & 0 & x_1^1 & y_1^1 & 1 & -x_3^1 y_3^2 & -y_3^1 y_3^2 \\ 0 & 0 & 0 & x_1^1 & y_1^1 & 1 & -x_4^1 y_4^2 & -y_4^1 y_4^2 \end{bmatrix} \times \begin{bmatrix} h_{11} \\ h_{12} \\ h_{13} \\ h_{21} \\ h_{22} \\ h_{23} \\ h_{31} \\ h_{32} \end{bmatrix} = \begin{bmatrix} x_1^2 \\ x_2^2 \\ x_3^2 \\ x_4^2 \\ y_1^2 \\ y_2^2 \\ y_3^2 \\ y_4^2 \end{bmatrix}$$

Ainsi, les coordonnées d'une tâche identifiée dans une surface d'observation représentée dans une première image, par exemple l'image 600, peuvent être projetées dans la surface d'observation correspondante d'une seconde image, par exemple l'image 605, pour permettre une comparaison de ces coordonnées avec celles de la tâche correspondante de la surface d'observation représentée dans la seconde image.

De même, les coordonnées d'une tâche identifiée dans une surface d'observation représentée dans une première image peuvent être projetées dans la surface d'observation correspondante d'une troisième image, par exemple l'image 610, pour permettre une comparaison de ces coordonnées avec celles de la tâche correspondante de la surface d'observation représentée dans la troisième image.

Selon d'autres modes de réalisation, la référence visuelle est utilisée pour définir les points devant être utilisés pour effectuer la projection, il peut s'agir de points distincts de la référence visuelle.

Il est également observé ici que la référence visuelle peut être utilisée pour identifier la surface d'observation du dispositif de test si les positions de cette dernière et de la référence visuelle sont prédéterminées.

Il est noté ici que l'analyse d'une image peut être perturbée par l'analyse d'artefacts tels que des poussières déposées sur l'enveloppe de protection 140. Dans ce cas, plusieurs images du dispositif de test peuvent être obtenues sensiblement au même instant, selon des points de vue différents pour détecter les artefacts afin de pouvoir les ignorer.

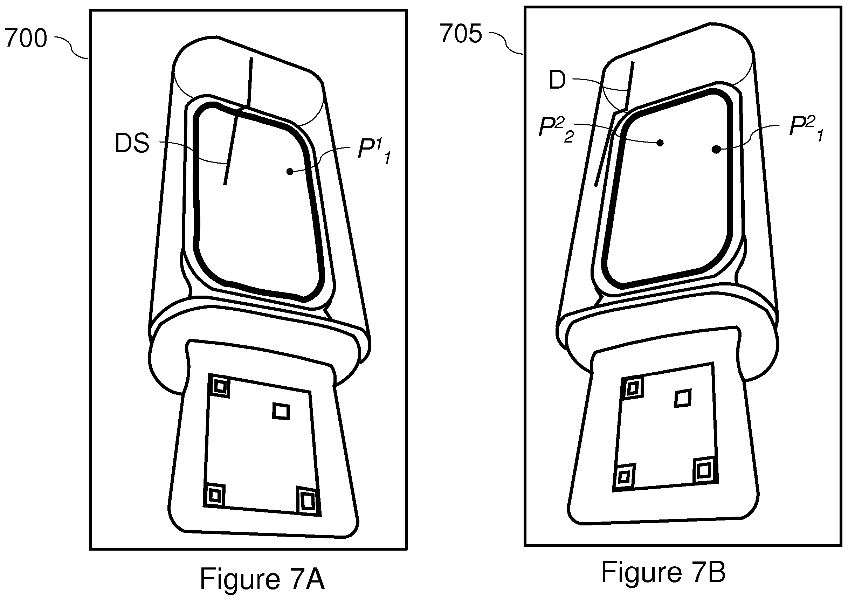

Les **figures 7A et 7B** illustrent un exemple de correction d'images selon un mode de réalisation particulier, entre une première image 700 et une deuxième image 705, respectivement.

Une telle correction peut être effectuée suite à la détection d'une suspicion de défaut dans une image ou dans une séquence d'images, par exemple lorsqu'un élément non attendu est détecté (en raison, par exemple, de sa forme, sa couleur, sa taille ou de son évolution dans le temps).

Les figures 7A et 7B montrent deux images représentant un même dispositif de test observé selon deux points de vue différents à deux instants proches l'un de l'autre. L'image représentée sur la figure 7B a ici été prise suite à la détection d'un défaut potentiel sur l'image représentée sur la figure 7A.

Plus précisément, l'analyse de la surface d'observation du dispositif de test représenté sur la figure 7A permet d'identifier une tâche $P^1_1$ et de détecter un défaut potentiel DS en raison, par exemple, de sa forme et de sa couleur.

Pour déterminer s'il s'agit d'un défaut ou d'une réaction particulière dans la zone de test, une autre image est obtenue depuis un point de vue différent, ici l'image représentée sur la figure 7B. Cette image est acquise dès que possible après l'obtention de l'image sur laquelle un défaut potentiel est détecté. A ces fins, l'application peut, après la détection d'un défaut potentiel, alerter l'utilisateur et lui suggérer l'acquisition d'une nouvelle image pour lever le doute.

L'analyse de la surface d'observation du dispositif de test représenté sur la figure 7B permet d'identifier les tâches $P^2_1$ et $P^2_2$ mais ne révèle aucun défaut potentiel. Il peut donc en être déduit que le défaut potentiel identifié sur la surface d'observation du dispositif de test représenté sur la figure 7A est un défaut qui peut être ignoré.

Selon des modes de réalisation, une analyse de la zone située autour de la surface d'observation du dispositif de test représenté sur l'image utilisée pour déterminer s'il s'agit d'un défaut ou non peut être effectuée pour identifier le défaut potentiel identifié précédemment. L'identification de ce défaut potentiel sur l'image de la figure 7B, en dehors de la surface d'observation, confirme qu'il s'agit d'un défaut lié à l'enveloppe de protection 140. A titre d'illustration, le défaut présenté sur la figure 7 est une griffure ou une marque sur la surface extérieure de l'enveloppe de protection 140. Il pourrait également s'agir, par exemple, d'une gouttelette de condensation sur la surface intérieure de l'enveloppe de protection 140.

Comme illustré sur les figures 7A et 7B, le changement de point de vue permet en outre de révéler, sur l'image de la figure 7B, la tâche $P^2_2$ qui était masquée sur l'image de la figure 7B.

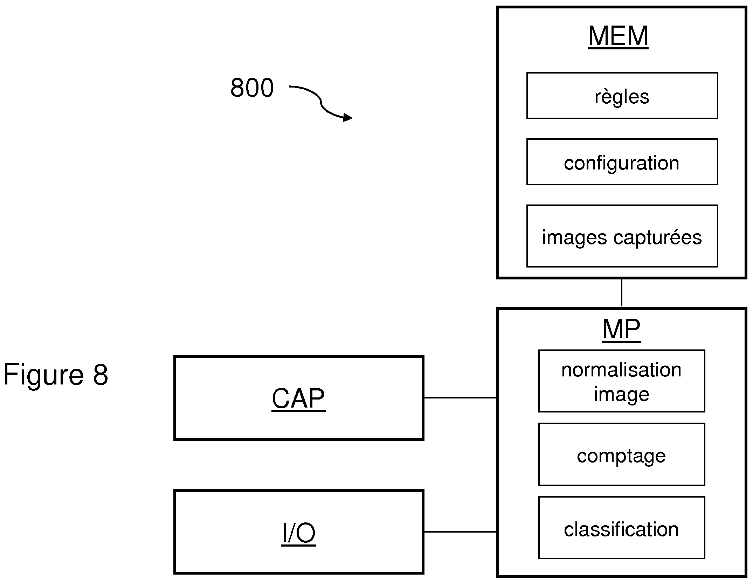

La **figure 8** représente un exemple d'une unité d'analyse 800 pouvant être utilisée par un système d'analyse selon un mode de réalisation particulier. L'unité d'analyse 800, par exemple un smartphone, comprend ici un capteur d'image noté CAP, une mémoire notée MEM, un micro-processeur noté MP et un module d'entrée/sortie noté I/O. Ces différents éléments sont ici contrôlés par une application exécutée sur l'unité d'analyse.

Le capteur d'image CAP est capable de prendre des images du dispositif de test 100 avec au moins une portion de la surface d'observation 120B et la référence visuelle 130 avec, de préférence, une résolution permettant d'analyser la surface d'observation selon le type de test à effectuer. A titre d'illustration, la capture d'images est effectuée par un utilisateur sur recommandation de l'application. Une image capturée est ensuite transférée au microprocesseur MP pour traitement.

La mémoire MEM peut être utilisée pour stocker l'application ainsi que des images capturées. Elle peut également être utilisée pour stocker, dans l'application ou de façon indépendante, des règles de comptage, de classification et de détermination de défaut, des règles d'incubation et des règles de validation des résultats. Alternativement, ces règles peuvent être stockées dans un système distant. La mémoire MEM peut, par exemple, être mise à jour par l'utilisateur final, par un laboratoire, par le développeur du logiciel ou par le fabricant du dispositif 100.

Le micro-processeur MP reçoit une image acquise par le capteur d'image CAP. Un premier test est, de préférence, effectué pour déterminer si l'image acquise est exploitable, par exemple si elle est suffisamment nette, si elle comprend une représentation de la référence visuelle et au moins une partie de la surface d'observation. La référence visuelle est ensuite, de préférence, analysée, par exemple pour accéder à et/ou vérifier des caractéristiques du dispositif de test 100, par exemple s'il n'est pas périmé et/ou s'il est compatible avec les conditions des tests envisagés. Des caractéristiques de l'unité d'analyse 700 peuvent ensuite être obtenues, par exemple des données de géolocalisation, une référence de temps (instant présent), etc.

Dans une étape suivante, le micro-processeur MP procède à une étape de normalisation d'une image acquise selon la référence visuelle. Comme décrit précédemment, une telle étape peut notamment consister en la définition d'un repère particulier dans lequel sont exprimées les coordonnées des points analysés ou dans la projection de l'image (ou de certains points de l'image) selon la référence visuelle et selon une autre image.

L'image acquise est ensuite analysée, selon la normalisation déterminée, afin, par exemple, d'identifier des tâches et de les comparer à des tâches identifiées dans une étape précédente.

Enfin, un module d'entrée/sortie I/O permet à un utilisateur de contrôler l'unité d'analyse, par exemple pour capturer des images, et de saisir des informations, par exemple des commentaires. Ce module permet également d'adresser des notifications à l'utilisateur, par exemple pour lui demander d'effectuer des actions telles que capturer une image, l'avertir d'actions à venir, par exemple le prévenir qu'il conviendra de capturer une nouvelle image dans un temps de n minutes ou n heures (n étant typiquement déterminé par l'application selon la nature des tests à effectuer), ou lui donner des indications ou informations, par exemple des indications relatives aux conditions de test et/ou au dispositif de test. Le module d'entrée/sortie I/O peut également être utilisé pour échanger des données avec un système distant, par exemple un serveur, par exemple pour transmettre des résultats de test. De tels échanges peuvent être prédéterminés et/ou peuvent être masqués vis-à-vis de l'utilisateur.

Selon des modes de réalisation, un ou plusieurs éléments de l'unité d'analyse sont absents ou ne sont pas utilisés, des éléments correspondant d'une autre unité étant utilisés. Ainsi, par exemple, un traitement d'images, par exemple une projection, pouvant demander des ressources de calcul importantes, il peut être déporté vers une unité distance, par exemple un serveur distant. L'unité d'analyse peut alors être utilisée comme capteur d'images et interface utilisateur.

Comme observé précédemment, l'application utilisée peut fournir des informations à l'utilisateur et le guider dans les tests à effectuer, par exemple pour lui indiquer comment obtenir un échantillon et le mettre en contact avec la zone de test du dispositif de test, quelles sont les conditions de tests, par exemple le temps et température d'incubation, l'assister dans la prises d'images, par exemple à l'aide d'indications d'alignement ou d'éclairage. Des exemples d'images peuvent également lui être présentés pour l'aider.

La **figure 9** illustre des étapes d'un exemple de procédé d'analyse selon un mode de réalisation. Le procédé d'analyse 900 comprend ici les étapes 901 à 909. Il peut être mis en oeuvre, par exemple, à l'aide du système 800 illustré sur la figure 8, par exemple sous la forme d'une application.

L'étape 901 a pour objet la capture d'une image du dispositif de test 100. A ces fins, une notification peut être adressée à un utilisateur, après que ce dernier ait lancé l'application ou qu'il ait lancé un nouveau test, afin qu'il capture une image du dispositif de test. La première image capturée peut être obtenue avant qu'un échantillon ne soit présenté à la zone de test du dispositif de test, par exemple pour obtenir une image de référence du dispositif de test, juste après qu'un échantillon soit présenté à la zone de test du dispositif de test pour avoir une première image de début de test ou après un certain temps d'incubation.

Da façon optionnelle, sur recommandation ou non de l'application, l'utilisateur peut prendre d'autres images, par exemple des images représentatives de l'environnement de test ou de la source de l'échantillon.

Un test de qualité est avantageusement effectué pour vérifier la qualité de l'image acquise, par exemple sa netteté et/ou la présence d'une représentation d'au moins une partie de la surface d'observation. Si la qualité n'est pas suffisante, par exemple au regard d'un critère prédéterminé, il peut être demandé à l'utilisateur de capturer une autre image.

Selon des modes de réalisation, l'image acquise est stockée avec des informations supplémentaires (métadonnées), telles qu'un identifiant de l'image, un identifiant de l'unité d'analyse utilisée pour capturer l'image, un identifiant de l'utilisateur ayant capturé l'image, une indication de la source de l'échantillon, une indication de température de l'environnement dans lequel se déroule le test, une information de géolocalisation telle que des données GPS (sigle de *global positioning system* en terminologie anglo-saxonne) et/ou une indication de date et heure de capture de l'image.

Dans une étape suivante (étape 902), une première analyse de l'image acquise est effectuée pour identifier une référence visuelle ayant un format prédéterminé (par exemple un QR code ou un code-barres), à l'aide d'un algorithme standard. L'identifiant correspondant à la référence visuelle peut être utilisé pour obtenir des données associées à cet identifiant dans une ou plusieurs bases de données. Comme décrit précédemment, de telles données sont, par exemple, un fabricant, un protocole de test et/ou une date de validité. Un lien est par ailleurs établi entre cet identifiant, l'image acquise et, de préférence, au moins certaines des informations supplémentaires ou métadonnées visées ci-dessus. L'image et ces informations supplémentaires peuvent notamment être stockées dans la ou les bases de données décrites ci-dessus en lien avec l'identifiant.

Selon l'exemple illustré, un test est ensuite effectué pour déterminer si la référence visuelle est valide, par exemple si son format est conforme au format attendu et/ou si elle définit un identifiant attendu (étape 903). En particulier, il peut être vérifié ici que l'identifiant correspondant à la référence visuelle est valide, c'est-à-dire, par exemple, s'il correspond à un dispositif de test non périmé et correspondant aux tests à effectuer. Il peut également être vérifié que la zone de test est bien représentée dans l'image ou qu'il n'y a, a priori, pas d'artefact.

Dans la négative, il peut être proposé à l'utilisateur de poursuivre ou non l'analyse en dépit de l'anomalie détectée ou d'effectuer une action correctrice (étape 904), par exemple prendre une nouvelle image. Selon des modes de réalisation particuliers, un message d'aide est adressé à l'utilisateur pour lui indiquer la nature de l'anomalie (e.g. « *dispositif expiré, continuer?* ») et/ou pour lui indiquer la raison de la défaillance et l'aider à la surmonter (e.g. « *proportion de la zone de test insuffisante : prendre une nouvelle image mieux cadrée* » ou « *potentiel artefact : prendre une nouvelle image sous un angle différent* »).

Si, au contraire, la référence visuelle est valide, l'image acquise peut être analysée. A ces fins, la référence visuelle est analysée pour déterminer une normalisation de l'image acquise (étape 905). Comme décrit précédemment, notamment en référence aux figures 5A à 5C et 6A à 6C, une telle normalisation peut notamment consister à déterminer un repère « normalisé » dans lequel sont exprimées les coordonnées des points étudiés ou à projeter l'image en cours d'analyse ou une partie de cette image en fonction du repère visuelle de cette image et du repère visuelle d'une image préalablement acquise (du même dispositif de test).

Dans une étape suivante, l'image est analysée (étape 906).

Cette analyse peut être conduite dans une unité d'analyse, par exemple un smartphone ou une tablette, ou dans un système, par exemple un serveur distant. L'analyse comprend l'identification et la caractérisation d'éléments sur une surface d'observation, typiquement l'identification et la caractérisation de tâches par leur couleur, leur forme et leur taille.

L'analyse comprend également, de préférence, pour la seconde image acquise et les suivantes, la comparaison des caractéristiques des éléments identifiés avec celles des éléments identifiés sur des surfaces d'observation d'images antérieures. Selon la normalisation utilisée, cette étape d'analyse comprend la comparaison de caractéristiques exprimées à partir d'un repère commun (ou « normalisé ») ou la comparaison de caractéristiques liées à une première image avec des caractéristiques d'une projection d'une seconde image (ou d'une partie de la seconde image). A ces fins, une ou plusieurs images antérieures (ou des caractéristiques obtenues par analyse de ces images) sont obtenues, en fonction de l'identifiant du dispositif de test. Cette analyse permet par exemple d'identifier des colonies de bactéries, de les compter et de les comparer entre des images successives.

Dans une étape suivante (étape 907), un test est effectué pour déterminer si les tests sont terminés ou non, c'est-à-dire si d'autres images doivent ou non être acquises et analysées. La fin du test peut être prédéterminée, par exemple selon une échelle de temps, ou liée à une observation, par exemple un nombre de colonies détectées.

Si les tests ne sont pas terminés, les étapes précédentes (étapes 901 à 907) sont répétées pour traiter une nouvelle image. Ces étapes sont répétées après un temps prédéterminé (étape 908), par exemple un temps d'incubation.

Dans le cas contraire, si les tests sont terminés, un rapport de test est, de préférence, établi (étape 909). Il contient, par exemple, des statistiques relatives aux éléments identifiés, à leurs caractéristiques et à leur évolution.

Il existe d'autres façons de mettre en oeuvre le procédé 900 illustré sur la figure 9. Par exemple, l'analyse d'une surface d'observation représentée dans une image peut commencer après la capture de cette dernière, sans attendre l'identification du dispositif de test. De même, dans des modes de réalisation, l'analyse des images peut être effectuée après qu'un grand nombre d'images, ou toutes les images aient été acquises. Il est également possible d'analyser une image tout en capturant une autre image.

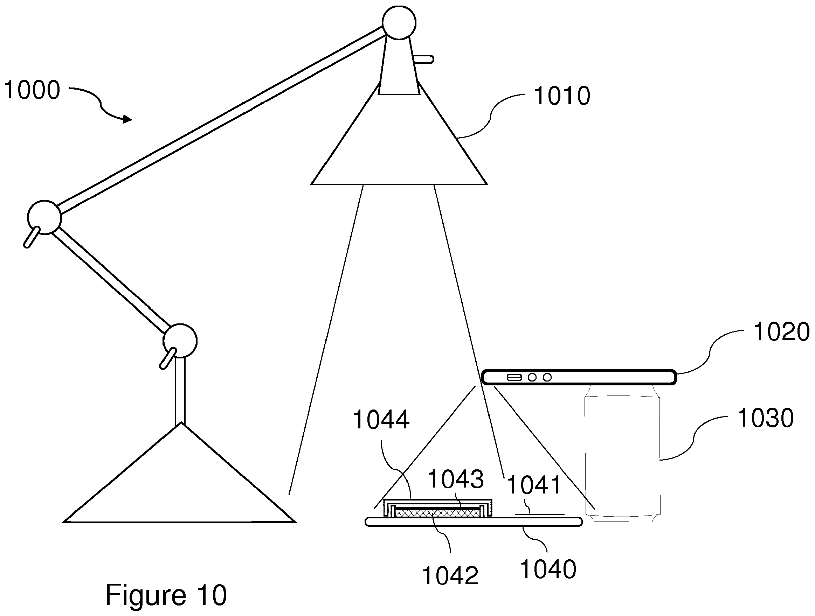

La **figure 10** représente un exemple de système d'analyse 1000 selon un mode de réalisation particulier. Le système 1000 est particulièrement adapté à une mise en oeuvre dans des environnements variés.

Selon l'exemple illustré ici, le système 1000 comprend une source de lumière artificielle 1010 (la lumière naturelle convient tout aussi bien, voire mieux que la lumière artificielle, mais n'est pas toujours disponible en quantité suffisante pour une bonne prise de vue), par exemple une lampe de bureau, une unité d'analyse 1020, par exemple un smartphone, un support 1030 pour l'unité d'analyse, par exemple une canette de boisson, et un dispositif de test 1040.

Comme illustré, vu de profil, le dispositif 1040 comprend une référence visuelle 1041, une zone de test 1042 et une surface d'observation 1043 protégée par une enveloppe de protection 1044.

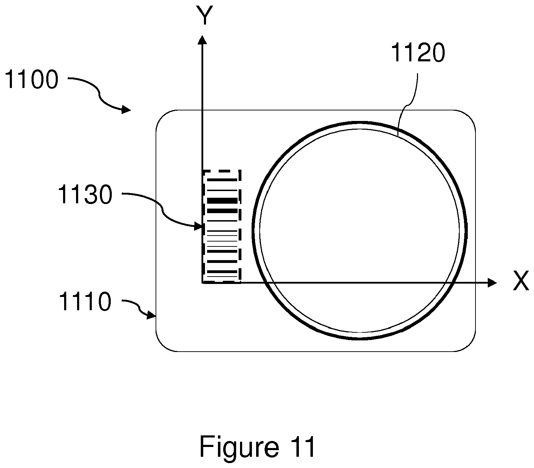

La **figure 11** représente une vue de face d'un dispositif de test 1100 selon un autre mode de réalisation. Le dispositif de test 1100 comprend ici un support 1110, une zone de test 1120 et une référence visuelle 1130. Selon cet exemple, la référence visuelle 1130 est un code-barres dont le format peut être utilisé pour définir un repère dans lequel peuvent être exprimées les coordonnées d'éléments détectés sur la surface d'observation de la zone de test 1120. Dans cet exemple, l'axe des abscisses est défini comme étant le bord inférieur du code-barres et l'axe des ordonnées comme étant le bord gauche du code-barres, l'origine du repère étant le coin en bas à gauche du code-barres (lorsque le dispositif est vu de face, la zone de test étant placée à droite).

Le dispositif de test 1100 peut être utilisé dans le cadre des systèmes d'analyse et procédés d'analyse décrits précédemment.

Naturellement, pour satisfaire des besoins spécifiques, une personne compétente dans le domaine de l'invention pourra appliquer des modifications dans la description précédente. La présente invention ne se limite pas aux formes de réalisation décrites, d'autres variantes et combinaisons de caractéristiques sont possibles.

La présente invention a été décrite et illustrée dans la présente description détaillée en référence aux figures jointes. Toutefois, la présente invention ne se limite pas aux formes de réalisation présentées. D'autres variantes et modes de réalisation peuvent être déduits et mis en œuvre par la personne compétente dans le domaine de l'invention à la lecture de la présente description et des figures annexées.

Dans les revendications, les termes « comprendre » ou « comporter » n'excluent pas d'autres éléments ou d'autres étapes. L'article indéfini « un » n'exclut pas le pluriel. Un seul processeur ou plusieurs autres unités peuvent être utilisés pour mettre en oeuvre l'invention. Les différentes caractéristiques présentées et/ou revendiquées peuvent être avantageusement combinées. Leur présence dans la description ou dans des revendications dépendantes différentes n'exclut pas, en effet, la possibilité de les combiner. Les signes de référence ne sauraient être compris comme limitant la portée de l'invention. Les limites de l'invention sont définies par les revendications jointes.

**Revendications**

1. Procédé d'analyse d'un dispositif de test, le procédé comprenant les étapes suivantes :

   - obtention d'au moins une image d'un dispositif de test (100, 1040, 1100), l'image comprenant au moins une représentation d'au moins une portion d'une surface d'observation (120B, 1043) et une référence visuelle (130, 1041, 1030) ayant un format prédéterminé ;
   - identification d'un dispositif de test en fonction de la référence visuelle ;
   - identification d'au moins un élément graphique dans la au moins une portion de la surface d'observation, ledit au moins un élément graphique résultant d'un test biologique ou chimique ;
   - détermination d'au moins une caractéristique géométrique de l'au moins un élément graphique identifié en fonction d'au moins la référence visuelle, l'au moins une caractéristique géométrique comprenant au moins une taille ou une forme ; et
   - analyse de la au moins une portion de la surface d'observation en fonction d'une identification du dispositif de test et d'au moins une caractéristique géométrique déterminée,

   l'étape d'analyse comprenant une étape de comparaison de la au moins une caractéristique géométrique déterminée avec au moins une caractéristique géométrique d'un élément graphique identifié dans une portion d'une surface d'observation représentée dans une autre image du même dispositif de test.

2. Procédé selon la revendication 1, comprenant en outre une étape de notification d'obtention d'une nouvelle image, la notification étant notifiée en fonction d'une identification du dispositif de test déterminée à partir d'une image obtenue précédemment.

3. Procédé selon la revendication 2, selon lequel l'étape de notification comprend une étape d'obtention d'une information de test, l'information de test étant obtenue en fonction de l'identification du dispositif de test déterminée à partir de l'image obtenue précédemment.

4. Procédé selon l'une quelconque des revendications 1 à 3, comprenant en outre une étape de mémorisation de l'image reçue et de l'identification du dispositif de test déterminée à partir de l'image obtenue.

**5.** Procédé selon l'une quelconque des revendications 1 à 4, selon lequel la au moins une caractéristique géométrique comprend en outre des coordonnées.

**6.** Programme d'ordinateur comprenant des instructions adaptées à la mise en œuvre de chacune des étapes du procédé selon l'une quelconque des revendications 1 à 5 lorsque ledit programme est exécuté sur un ordinateur.

**7.** Système d'analyse d'un dispositif de test comprenant des moyens adaptés à la mise en œuvre de chacune des étapes du procédé selon l'une quelconque des revendications 1 à 5.

**8.** Système selon la revendication 7, comprenant un dispositif de capture d'image, de correction d'image et d'analyse d'images de type smartphone.

**9.** Système selon la revendication 7, comprenant un premier dispositif de capture d'image et un second dispositif d'analyse d'images, distinct du premier dispositif.

**Patentansprüche**

**1.** Verfahren zur Analyse einer Testvorrichtung, wobei das Verfahren die folgenden Schritte umfasst:

- Erhalten mindestens eines Bildes von einer Testvorrichtung (100, 1040, 1100), wobei das Bild mindestens eine Darstellung von mindestens einem Abschnitt einer Beobachtungsoberfläche (120B, 1043) und eine visuelle Referenz (130, 1041, 1030) in einem vorher festgelegten Format umfasst;
- Identifizieren einer Testvorrichtung in Abhängigkeit von der visuellen Referenz;
- Identifizieren mindestens eines graphischen Elements in dem mindestens einen Abschnitt der Beobachtungsoberfläche, wobei das mindestens eine graphische Element aus einem biologischen oder chemischen Test resultiert;
- Bestimmen mindestens eines in Abhängigkeit von mindestens der visuellen Referenz identifizierten geometrischen Merkmals des mindestens einen graphischen Elements, wobei das mindestens eine geometrische Merkmal mindestens eine Größe oder eine Form umfasst; und
- Analysieren des mindestens einen Abschnitts der Beobachtungsoberfläche in Abhängigkeit von einer Identifizierung der Testvorrichtung und von mindestens einem bestimmten geometrischen Merkmal,

wobei der Schritt des Analysierens einen Schritt des Vergleichens des mindestens einen bestimmten geometrischen Merkmals mit mindestens einem geometrischen Merkmal eines graphischen Elements umfasst, das in einem Abschnitt einer Beobachtungsoberfläche identifiziert ist, der in einem anderen Bild derselben Testvorrichtung dargestellt ist.

**2.** Verfahren nach Anspruch 1, umfassend ferner einen Schritt des Mitteilens des Erhalts eines neuen Bildes, wobei die Mitteilung in Abhängigkeit von einer ausgehend von einem zuvor erhaltenen Bild bestimmten Identifizierung der Testvorrichtung mitgeteilt wird.

**3.** Verfahren nach Anspruch 2, wobei der Schritt des Mitteilens einen Schritt des Erhaltens einer Testinformation umfasst, wobei die Testinformation in Abhängigkeit von der ausgehend von dem zuvor erhaltenen Bild bestimmten Identifizierung der Testvorrichtung mitgeteilt wird.

**4.** Verfahren nach einem der Ansprüche 1 bis 3, umfassend ferner einen Schritt des Speicherns des empfangenen Bildes und der ausgehend von dem erhaltenen Bild bestimmten Identifizierung der Testvorrichtung.

**5.** Verfahren nach einem der Ansprüche 1 bis 4, wobei das mindestens eine geometrische Merkmal ferner Koordinaten umfasst.

**6.** Rechnerprogramm, das für die Durchführung von jedem der Schritte des Verfahrens nach einem der Ansprüche 1 bis 5 geeignete Befehle umfasst, wenn das Programm auf einem Rechner ausgeführt wird.

**7.** System zur Analyse einer Testvorrichtung, das für die Durchführung von jedem der Schritte des Verfahrens nach einem der Ansprüche 1 bis 5 geeigneten Mittel umfasst.

8. System nach Anspruch 7, welches eine Bilderfassungs-, Bildkorrektur- und Bildanalysevorrichtung vom Typ Smartphone umfasst.

9. System nach Anspruch 7, welches eine erste Bilderfassungsvorrichtung und eine zweite Bildanalysevorrichtung umfasst, die sich von der ersten Vorrichtung unterscheidet.

**Claims**

1. A method of analyzing a test device, the method comprising the following steps:

   - obtaining at least one image of a test device (100, 1040, 1100), the image comprising at least one representation of at least one portion of an observation surface (120B, 1043) and a visible reference (130, 1041, 1030) having a predetermined format;
   - identifying a test device according to the visible reference;
   - identifying at least one graphical element in the at least one portion of the observation surface, said at least one graphical element resulting from a biological or chemical test;
   - determining at least one geometrical feature of a graphical element identified according to at least the visible reference, the at least one geometrical feature comprising at least a size or a shape; and
   - analyzing the at least one portion of the observation surface according to an identification of the test device and at least one determined geometrical feature,

   the analysis step comprising a step of comparing the at least one determined geometrical feature with at least one geometrical feature of a graphical element identified in a portion of an observation surface represented in another image of the same test device.

2. The method according to claim 1, further comprising a notification step giving notification that a new image has been obtained, the notification being given according to an identification of the test device determined from an image obtained previously.

3. The method according to claim 2, wherein the notification step comprises a step of obtaining a test information item, the test information item being obtaining according to the test device identification determined from the image obtained previously.

4. The method according to any one of claims 1 to 3, further comprising a step of storing the received image and the test device identification determined from the obtained image.

5. The method according to any one of claims 1 to 4, wherein the at least one geometrical feature further comprises coordinates.

6. A computer program comprising instructions adapted for carrying out each of the steps of the method according to any one of claims 1 to 5 when said program is executed on a computer.

7. A system for analyzing a test device comprising means configured for carrying out each of the steps of the method according to any one of claims 1 to 5.

8. The system according to claim 7, comprising a device of smartphone type for image capture, image correction and analysis of images.

9. The system according to claim 7, comprising a first device for image capture and a second device for analysis of images, distinct from the first device.

10

14

12

22

20

16

24

Figure 1
(Art Antérieur)

Figure 2A

Figure 2B

100

110B

130

110

110C

110A

120A

120B

120

140

Z

Y

Figure 3

100

$O_1(t_0)$

**Figure 4A**

100

$O_1(t_1)$     $O_2(t_1)$

**Figure 4B**

100

$O_3(t_3)$

$O_1(t_3)$     $O_2(t_3)$

**Figure 4C**

100

$O_3(t_4)$

$O_1(t_4)$     $O_2(t_4)$

**Figure 4D**

Y

130

$C_3$

$C_4$

$C_1$

$C_2$

X

**Figure 5A**

Figure 5C

Figure 5B

Figure 6A

Figure 6B

Figure 6C

EP 3 557 528 B1

Figure 7B

Figure 7A

Figure 8

800

**MEM**

règles

configuration

images capturées

**MP**

normalisation image

comptage

classification

CAP

I/O

Figure 9

Figure 10

Figure 11

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014080212 A
- WO 2016172527 A
- US 7298885 B

**Littérature non-brevet citée dans la description**

- Perspective Correction Methods for Camera-Based Document Analysis. **L. JAGANNATHAN ; C. V. JA-WAHAR.** Proceedings of First International Workshop on Camera Based Document Analysis and Récognition. Août 2005, 148-154